# EUROPEAN PATENT APPLICATION

(11) **EP 4 212 185 A1**
(43) Date of publication of application: **19.07.2023**
(21) Application number: 21866331.8
(22) Date of filing: 22.06.2021
(51) Int. Cl.: A61L 2/10

(54) **STERILIZATION SYSTEM, STERILIZATION DEVICE, CONTROL DEVICE, CONTROL METHOD, AND CONTROL PROGRAM**

(30) Priority: 14.09.2020 JP 2020153592
(71) Applicant: Minebea Mitsumi Inc., Nagano 389-0293 (JP)
(72) Inventor: IMAMURA, Shogo, Kitasaku-gun, Nagano 389-0293 (JP); SAGAWA, Yoshihiro, Kitasaku-gun, Nagano 389-0293 (JP)
(74) Representative: Zabel, Julia Elisabeth
(86) International application number: PCT/JP2021/023613
(87) International publication number: WO 2022/054370

(57) **Abstract**

A sterilization system of an embodiment includes a light source unit (21), a horizontal angle adjustment unit (4) and/or a vertical angle adjustment unit (3), and a controller (5). The light source unit (21) emits ultraviolet rays in a radiating form. The horizontal angle adjustment unit (4) adjusts a horizontal angle of the light source unit (21) by causing rotation of the light source unit (21) in a horizontal direction. The vertical angle adjustment unit (3) adjusts a vertical angle of the light source unit (21) by causing rotation of the light source unit (21) in a vertical direction. The controller (5) captures a target of sterilization from a camera image, controls rotation of the horizontal angle adjustment unit (4) and/or the vertical angle adjustment unit (3), and irradiates the target with the ultraviolet rays from the light source unit (21).

## Description

### Technical Field

The present invention relates to a sterilization system, a sterilizer, a controller, a control method, and a control program.

### Background Art

A sterilizer using ultraviolet ray (ultraviolet light) irradiation to sterilize bacteria without affecting human cells has been proposed and known (see, for example, Patent Document 1 and the like).

### Citation List

### Patent Literature

Patent Document 1: JP 6025756 B

### Summary of Invention

### Technical Problem

However, the known sterilizer performs sterilization on a certain target object to be sterilized, and does not correspond to sterilization for a wide range of space such as a room and factory.

On the other hand, sterilization target objects such as viruses, including COVID-19, and bacteria float in a space and adhere to a wall forming the space and the like. For the reason, there has been no choice but to take measures, and the implementation of the measures is limited due to the necessity of changing air conditioning equipment or due to a large workload, such as replacement of air in the space or inactivation by spraying an antiseptic solution. There has been a demand for easier improvement of the environment in the space.

The present invention has been made in view of the above, and an object of the present invention is to provide a sterilization system, a sterilizer, a controller, a control method, and a control program capable of easily sterilizing a wide range of sterilization target space.

### Solution to Problem

A sterilizer according to an aspect of the present invention to solve the problem described above and achieve the object includes a light source unit, a horizontal angle adjustment unit and/or a vertical angle adjustment unit, and a controller. The light source unit emits ultraviolet rays in a radiating form. The horizontal angle adjustment unit adjusts a horizontal angle of the light source unit by causing rotation of the light source unit in a horizontal direction. The vertical angle adjustment unit adjusts a vertical angle of the light source unit by causing rotation of the light source unit in a vertical direction. The controller captures a target of sterilization from a camera image, controls rotation of the horizontal angle adjustment unit and/or the vertical angle adjustment unit, and irradiates the target with the ultraviolet rays from the light source unit.

A sterilization system according to an aspect of the present invention can easily sterilize a wide range of sterilization target space.

### Brief Description of Drawings

FIG. 1 is a block diagram illustrating an example of a configuration of a sterilizer and the like according to an embodiment.
FIG. 2 is a diagram illustrating an example of a configuration in a case where a plurality of sterilizers are included.
FIG. 3 is a perspective view illustrating an example of a mechanical configuration of an example of the sterilizer.
FIG. 4 is a perspective view illustrating another example of a mechanical configuration of an example of the sterilizer.
FIG. 5 is a diagram illustrating an example of a hardware configuration of a control unit of a controller, a control unit of a terminal, or a control unit of a controller.
FIG. 6 is a diagram illustrating an example of a functional configuration of the control unit of the controller, the control unit of the terminal, or the control unit of the controller.
FIG. 7 is a flowchart illustrating an example of control by the control unit of the controller, the control unit of the terminal, or the control unit of the controller.
FIG. 8 is a flowchart illustrating another example of the control by the control unit of the controller, the control unit of the terminal, or the control unit of the controller.

### Description of Embodiments

A sterilization system, a sterilizer, a controller, a control method, and a control program according to an embodiment will be described below with reference to the drawings. Note that the present invention is not limited to the embodiment described above. Furthermore, the dimensional relationships between elements, proportions of the elements, and the like in the drawings may differ from reality. The drawings may each include parts having mutually different dimensional relationships and proportions. Furthermore, the contents described in one embodiment or modified example are applied in principle to other embodiments or modified examples.

### Device Configuration, System Configuration

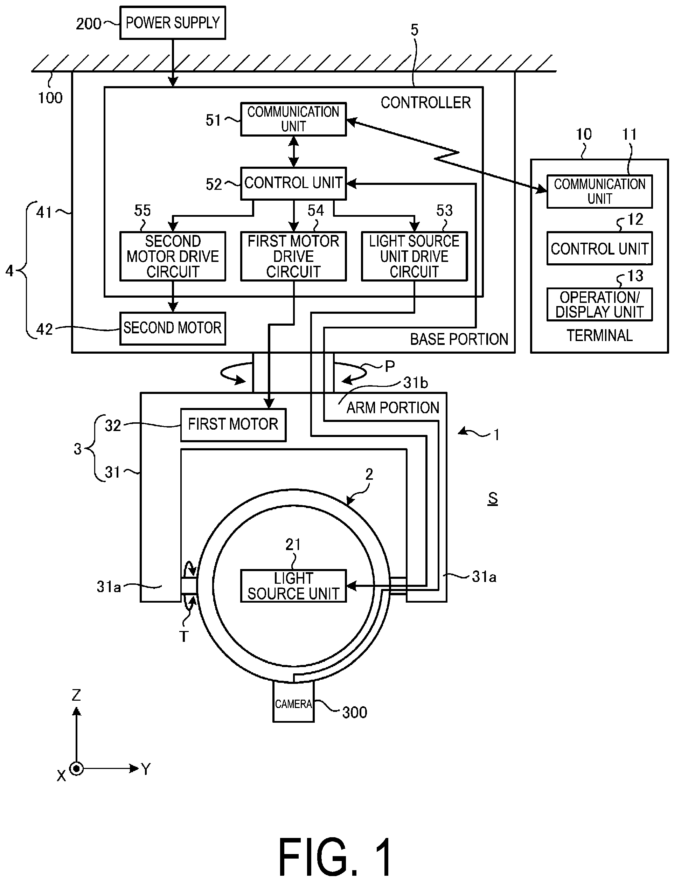

FIG. 1 is a block diagram illustrating an example of a configuration of a sterilizer 1 and the like according to an embodiment. In FIG. 1, the sterilizer 1 has a base portion 41 attached to a ceiling wall 100 forming a ceiling, for example, and includes: a main body portion 2 having a light source unit 21; a vertical angle adjustment unit 3 and a horizontal angle adjustment unit 4 rotatably supporting the main body portion 2; and a controller 5.

An X direction in FIG. 1 is a horizontal direction and is a front and back direction of the sterilizer 1A. A Y direction is a horizontal direction and is a width direction of the sterilizer 1A. A Z direction is a vertical direction orthogonal to the horizontal direction and is an upward and downward direction of the sterilizer 1A. In the present embodiment, the Z direction is parallel to the vertical direction of a sterilization target space S, but is not limited to the vertical direction, and the Z direction may be inclined with respect to the vertical direction or the Z direction may be parallel to a direction orthogonal to the vertical direction.

The controller 5 controls the sterilizer 1. The controller 5 controls the light source unit 21, the vertical angle adjustment unit 3, and the horizontal angle adjustment unit 4, and is contained in an internal space of the base portion 41. The controller 5 includes a communication unit 51, a control unit 52, a light source unit drive circuit 53, a first motor drive circuit 54, and a second motor drive circuit 55. Based on electric power supplied from a power supply 200, the controller 5 performs turning ON/OFF of emission of ultraviolet rays by the light source unit 21, adjustment of the irradiation intensity, adjustment of a vertical angle of the light source unit 21 by the vertical angle adjustment unit 3, adjustment of a horizontal angle of the light source unit 21 by the horizontal angle adjustment unit 4, and the like. The hardware configuration of the controller 5 is the same as the hardware configuration of a general controller, and will be described in detail below. The power supply 200 is a commercial power supply, a generator, a battery, or the like.

The communication unit 51 transmits and receives information to and from the terminal 10 carried by a user, and, for example, receives operation information from the terminal 10, and transmits a status of the sterilizer 1, an irradiation status (for example, ON/OFF, irradiation intensity) of the light source unit 21, and an orientation status (for example, the vertical angle and the horizontal angle) of the light source unit 21 to the terminal 10. The communication unit 51 performs short-range wireless communications using Wi-Fi (registered trademark), Bluetooth (registered trademark), or the like for example.

The control unit 52 performs irradiation control on the light source unit 21, drive control on a first motor 32, drive control on a second motor 42, and the like. Here, the irradiation control includes ON/OFF control and irradiation intensity control on the light source unit 21.

The light source unit drive circuit 53 supplies electric power to the light source unit 21 based on an irradiation control signal for the light source unit 21 from the control unit 52. When the light source unit 21 is of a type capable of changing the irradiation intensity of ultraviolet rays, the light source unit drive circuit 53 also adjusts the irradiation intensity. In addition, when the light source unit drive circuit 53 is of a type capable of changing (switching) the wavelength of the ultraviolet rays emitted by the light source unit 21, the light source unit drive circuit 53 also changes (switches) the wavelength. The light source unit drive circuit 53 is electrically connected to the control unit 52 and the light source unit 21. The light source unit drive circuit 53 is included in the controller 5, but is not limited to being included in the controller 5, and may be included in the light source unit 21. When the light source unit 21 is a light source combined with light sources of different wavelength ranges, adjustment is performed so that the irradiation intensity of each of the light sources can be adjusted.

The first motor drive circuit 54 supplies driving electric power to the first motor 32 based on a drive control signal for the first motor 32 from the control unit 52, to rotate the light source unit 21 in the vertical direction. The first motor drive circuit 54 is electrically connected to the control unit 52 and the first motor 32. The first motor drive circuit 54 is included in the controller 5 but is not limited to being included in the controller 5, and may be included in the first motor 32.

The second motor drive circuit 55 supplies driving electric power to the second motor 42 based on a drive control signal for the second motor 42 from the control unit 52, to rotate the light source unit 21 in the horizontal direction. The second motor drive circuit 55 is electrically connected to the control unit 52 and the second motor 42. The second motor drive circuit 55 is included in the controller 5 but is not limited to being included in the controller 5, and may be included in the second motor 42.

The user operates the terminal 10 to operate the sterilizer 1A, and to recognize the status of the sterilizer 1A. The terminal 10 includes a communication unit 11, a control unit 12, and an operation/display unit 13. The terminal 10 may be a dedicated terminal for the sterilizer 1A, or may be a general-purpose terminal such as a smartphone, tablet, and laptop computer. The hardware configuration of the terminal 10 is similar to the hardware configuration of a general terminal, and will be described in detail below.

The communication unit 11 transmits and receives information to and from the controller 5. The communication unit 11 performs short-range wireless communications using Wi-Fi (registered trademark), Bluetooth (registered trademark), or the like for example. Note that transmission and reception of information is not limited to wireless, and may be wired transmission and reception.

The control unit 12 controls the terminal 10 and outputs operation information to the communication unit 11 based on at least an operation on the operation/display unit 13 by the user. The control unit 12 also controls display by the operation/display unit 13. The control unit 12 is electrically connected to the communication unit 11 and the operation/display unit 13. Note that the control unit 12 can take over part of processing of the control unit 52 connected via the communication unit 11 on the terminal side and the communication unit 51 on the sterilizer 1 side.

The operation/display unit 13 is used for a remote operation of the sterilizer 1A in addition to a general operation and display in the terminal 10. Although not illustrated, the operation/display unit 13 includes, for example, a switch corresponding to turning ON/OFF of the ultraviolet rays, a switch corresponding to the irradiation intensity, a switch corresponding to adjustment of the vertical angle of the light source unit 21, and a switch corresponding to adjustment of the horizontal angle of the light source unit 21. Note that the switches may be a mechanical switch such as an energized/de-energized switch or a resistance change switch, or may be an electrical switch such as a touch panel.

A camera 300 is provided at the vicinity of the main body portion 2, and the control unit 52 analyzes an image signal from the camera 300 to recognize a target of the sterilization such as a person or a pet.

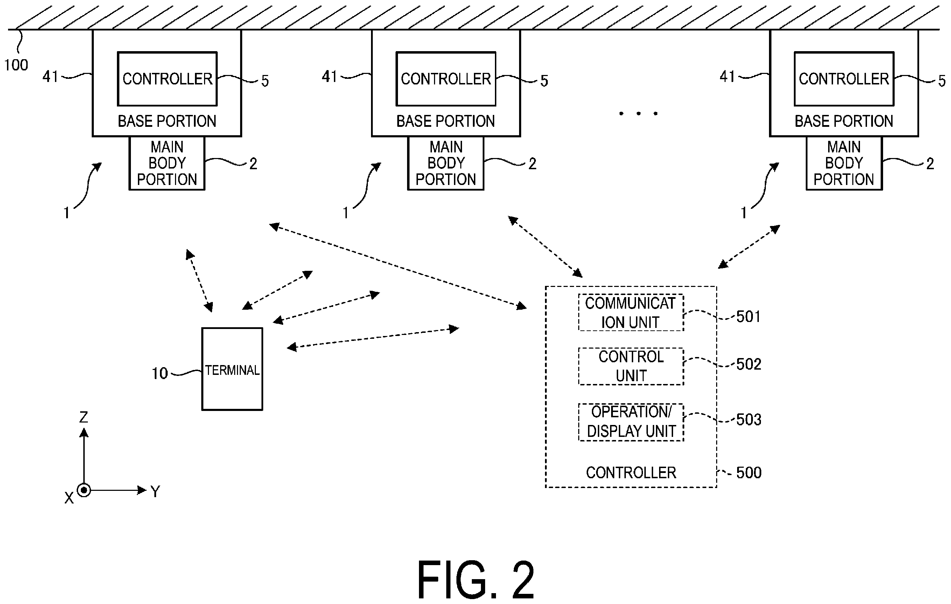

FIG. 2 is a diagram illustrating an example of a configuration in a case where a plurality of the sterilizers 1 are included. In FIG. 2, the plurality of sterilizers 1 are attached to the ceiling wall 100 forming a ceiling, for example. The controllers 5 of the respective sterilizers 1 perform control in an equal relationship, or one of the controllers 5 serves as a master to perform overall control. A controller 500 for the overall control may be provided separately from the controllers 5 of the sterilizers 1. The controller 500 includes a communication unit 501, a control unit 502, and an operation/display unit 503.

The communication unit 501 transmits and receives information to and from the controller 5 of each of the sterilizers 1. The communication unit 501 performs short-range wireless communications using Wi-Fi (registered trademark), Bluetooth (registered trademark), or the like for example. Note that transmission and reception of information is not limited to wireless, and may be wired transmission and reception.

The control unit 502 controls the controller 500 and outputs operation information to the communication unit 501 based on at least an operation on the operation/display unit 503 by the user or information set in advance. The control unit 502 is electrically connected to the communication unit 501 and the operation/display unit 503.

The operation/display unit 503 is used for a remote operation of each of the sterilizers 1 in addition to a general operation and display in the controller 500. Although not illustrated, the operation/display unit 503 includes, for example, a switch corresponding to turning ON/OFF of the ultraviolet rays, a switch corresponding to the irradiation intensity, a switch corresponding to adjustment of the vertical angle of the light source unit 21, a switch corresponding to adjustment of the horizontal angle of the light source unit 21, and a switch for setting various automatic operations. Note that the switches may be a mechanical switch such as an energized/de-energized switch or a resistance change switch, or may be an electrical switch such as a touch panel.

The terminal 10 communicates with the controller 5 of each of the sterilizers 1, with the controller 5 set to be the master, or with the controller 500, to correspond to various operations and status confirmation by the user. The terminal 10 can control each sterilizer 1 together with the controller 500 or in place of the controller 500.

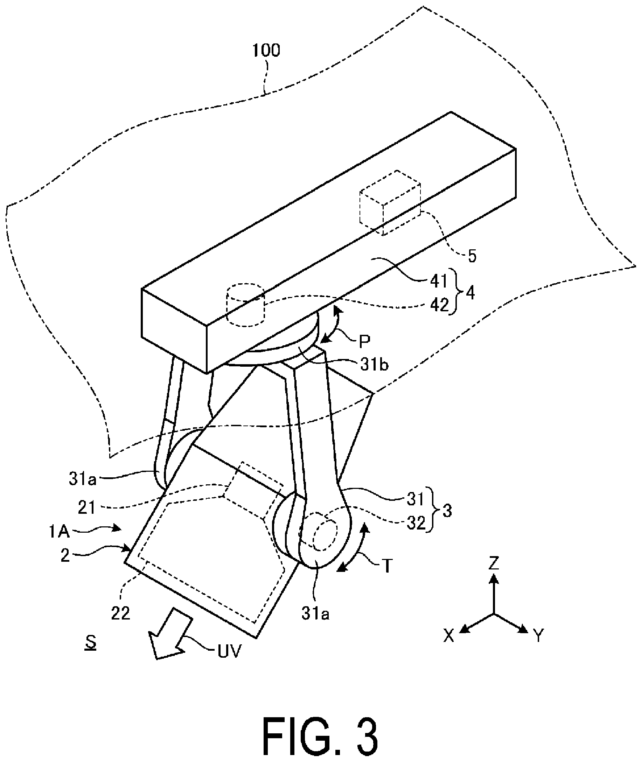

FIG. 3 is a perspective view illustrating an example of a mechanical configuration of an example of the sterilizer 1 (sterilizer 1A). As illustrated in FIG. 3, the sterilizer 1A irradiates the sterilization target space S with ultraviolet rays UV. The sterilizer 1A is attached to a wall forming a part of the sterilization target space S, that is, the ceiling wall 100 forming the ceiling in the present embodiment. Specifically, the sterilizer 1A is attached to the ceiling wall 100 via a rail or the like not illustrated, in a state of protruding from the ceiling wall 100 into the sterilization target space S. The sterilizer 1A includes the main body portion 2 with the light source unit 21 attached, the vertical angle adjustment unit 3, the horizontal angle adjustment unit 4, and the controller 5.

The light source unit 21 is attached to the main body portion 2. The main body portion 2 is formed in a substantially cylindrical shape, with an opening 22 formed at the sterilization target space S side in an axial direction and with the side opposite to the sterilization target space S closed. The main body portion 2 is supported, via a supporting shaft not illustrated, to be rotatable with respect to the vertical angle adjustment unit 3.

The light source unit 21 irradiates the sterilization target space S with the ultraviolet rays UV in a radiating form. The light source unit 21 is attached to a position opposing the opening 22 in the axial direction of the main body portion 2, in the internal space of the main body portion 2. The light source unit 21 irradiates the sterilization target space S with the ultraviolet rays UV through the opening 22. The ultraviolet rays UV emitted to the sterilization target space S by the light source unit 21 are electromagnetic waves having wavelengths different from the wavelength of visible light, and refer to electromagnetic waves having wavelengths of 190 nm to 230 nm in the present embodiment. Ultraviolet rays below 190 nm are absorbed by air, particularly oxygen in air. Thus, the amount of the ultraviolet rays reaching an object present in the sterilization target space S, for example, a human body, is greatly reduced, and the sterilization effect on the object is reduced. On the other hand, when the object present in the sterilization target space S is a human body, ultraviolet rays exceeding 230 nm may be absorbed by cells and damage DNA in the cells. The light source unit 21 of the present embodiment has, as the light source, a KrCl excimer lamp having a peak at the 222 nm for example. The ultraviolet rays UV in a radiating form may be emitted from the light source, or the ultraviolet rays of a radiating form may be obtained by a lens not illustrated from the ultraviolet rays UV in a linear form emitted from the light source.

The light source unit 21 is not limited to a light source emitting the ultraviolet rays UV in one wavelength range, and may be capable of switching between ultraviolet rays UV in different wavelength ranges. The light source unit 21 may be a combination of the light source emitting the ultraviolet rays UV, and a light source emitting electromagnetic waves, for example, visible light or infrared rays in a wavelength range different from the wavelength range of the ultraviolet rays UV. The light source unit 21 may also be a combination of a light source emitting ultraviolet rays in one wavelength range, and a light source emitting ultraviolet rays in a wavelength range different from the wavelength range. Specifically, the light source unit 21 may be a combination of a light source emitting ultraviolet rays having a wavelength of 190 nm to 230 nm, and a light source emitting ultraviolet rays having a wavelength of longer than 230 nm.

The vertical angle adjustment unit 3 rotates the light source unit 21 in the vertical direction Z, to adjust the vertical angle, that is, a tilt direction T. The vertical angle adjustment unit 3 includes an arm portion 31 and a first motor 32. The main body portion 2 is supported by the arm portion 31 to be rotatable in the vertical direction Z. The arm portion 31 is formed in a U-shape opening in the downward direction, and the main body portion 2 is supported, at both distal end portions 31a and 31a forming the opening, to be rotatable about a direction, and in the direction, the both distal end portions 31a and 31a oppose each other. The first motor 32 is a vertical actuator, and rotates the main body portion 2 with respect to the arm portion 31. The first motor 32 is attached to the arm portion 31 and rotates the main body portion 2 about the supporting shaft, that is, about the opposing direction, via a driving mechanism not illustrated. The light source unit 21 of the present embodiment is swung by the first motor 32 in a range of, for example, ±90 degrees when the downward direction is defined as 0 degrees. The vertical angle adjustment unit 3 is supported, via a supporting shaft not illustrated, to be rotatable with respect to the horizontal angle adjustment unit 4.

The horizontal angle adjustment unit 4 adjusts a horizontal angle, that is, adjusts a pan direction P by causing rotation of the light source unit 21 in the horizontal direction (the front and back direction X and the width direction Y). The horizontal angle adjustment unit 4 includes the base portion 41 and the second motor 42. The main body portion 2 is supported by the base portion 41 to be rotatable in the horizontal direction, via the arm portion 31. The base portion 41 is attached to the ceiling wall 100 and formed in a cubic shape with a longitudinal direction being in the front and back direction. On the base portion 41, a distal end portion 31b of the arm portion 31 in an upper direction side is supported so that the arm portion 31 is supported to be rotatable in the vertical direction Z. The second motor 42 is a horizontal actuator and rotates the arm portion 31 with respect to the base portion 41. The second motor 42 is attached to the base portion 41, and rotates the arm portion 31 about the supporting shaft, that is, the vertical direction, via a driving mechanism not illustrated, to rotate the main body portion 2. The light source unit 21 of the present embodiment is swung by the second motor 42 in a range of, for example, ±180 degrees when any of the horizontal directions is defined as 0 degrees.

In the above description, each actuator is a motor, but the motor is not limited to the actuator, and may be a hydraulic cylinder, a pneumatic cylinder, or the like.

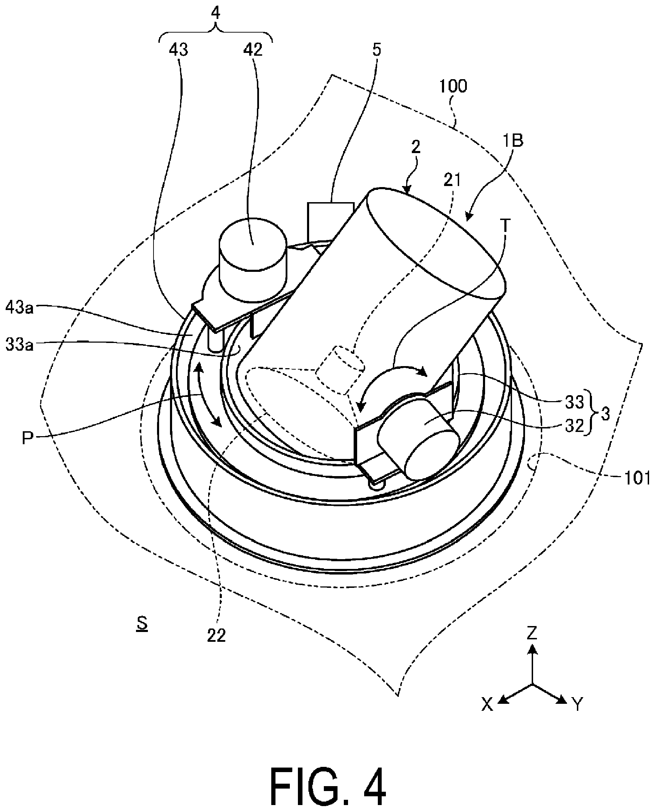

FIG. 4 is a perspective view illustrating an example of a mechanical configuration of another example of the sterilizer 1 (sterilizer 1B). In FIG. 4, the sterilizer 1B differs from the above-described sterilizer 1A in the state of attachment to the ceiling wall 100. Since the basic configuration of the sterilizer 1B is identical or substantially identical to the basic configuration of the sterilizer 1A, description of identical reference numerals will be omitted or simplified.

As illustrated in FIG. 4, the sterilizer 1B irradiates the sterilization target space S with ultraviolet rays UV. The sterilizer 1B is attached to the ceiling wall 100 forming a part of the sterilization target space S. Specifically, the sterilizer 1A is attached to the ceiling wall 100 while protruding from the ceiling wall 100 toward the side opposite to the sterilization target space S side, that is, while being buried in the ceiling wall 100. An opening 101 is formed at the ceiling wall 100, and the sterilizer 1B is attached with the sterilizer 1B, in particular, the opening 22 of the main body portion 2 exposed to the sterilization target space S through the opening 101. The sterilizer 1B includes the main body portion 2 with the light source unit 21 attached, the vertical angle adjustment unit 3, the horizontal angle adjustment unit 4, and the controller 5.

The light source unit 21 irradiating the sterilization target space S with the ultraviolet rays UV in a radiating form is attached to the main body portion 2. The main body portion 2 is formed in a substantially cylindrical shape, with an opening 22 formed at the sterilization target space S side in the axial direction and with the side opposite to the sterilization target space S side closed. The main body portion 2 is supported, via a supporting shaft not illustrated, to be rotatable with respect to the vertical angle adjustment unit 3.

The vertical angle adjustment unit 3 rotates the light source unit 21 in the vertical direction Z, to adjust the vertical angle, that is, a tilt direction T. The vertical angle adjustment unit 3 includes a sub frame portion 33 and the first motor 32. The main body portion 2 is supported by the sub frame portion 33 to be rotatable in the vertical direction Z. The sub frame portion 33, having an internal space 33a formed communicating with the outside through an opening in the upward and downward direction, is formed in a cylindrical shape with the axis in the upward and downward direction. In the internal space 33a of the sub frame portion 33, the main body portion 2 is supported to be rotatable about the horizontal direction. Specifically, in the sub frame portion 33, at least part of the main body portion 2 is accommodated in the internal space 33a, and the main body portion 2 is supported to be rotatable about the vertical direction. The first motor 32 is attached to the sub frame portion 33 and rotates the main body portion 2 about the supporting shaft, that is, about the opposing direction, via a driving mechanism not illustrated. The light source unit 21 of the present embodiment is swung by the first motor 32 in a range of, for example, -35 degrees to +45 degrees when the downward direction is defined as 0 degrees.

The horizontal angle adjustment unit 4 adjusts a horizontal angle, that is, adjusts a pan direction P by causing rotation of the light source unit 21 in the horizontal direction (the front and back direction X and the width direction Y). The horizontal angle adjustment unit 4 includes a base frame portion 43 and the second motor 42. The main body portion 2 is supported by the base frame portion 43 to be rotatable in the horizontal direction, via the sub frame portion 33. The base frame portion 43, with an internal space 43a formed communicating with the outside through an opening in the upward and downward direction, is attached to the ceiling wall 100 while opposing the opening 101 in the upward and downward direction, and is formed in a cylindrical shape with the axis in the upward and downward direction. In the internal space 43a of the base frame portion 43, the main body portion 2 is supported to be rotatable about the vertical direction. Specifically, in the base frame portion 43, at least part of the sub frame portion 33 is accommodated in the internal space 43a, and the sub frame portion 33 is supported to be rotatable about the vertical direction. The light source unit 21 of the present embodiment is swung by the second motor 42 in a range of, for example, +355 degrees when any of the horizontal directions is defined as 0 degrees.

In the above description, each actuator is a motor, but the motor is not limited to the actuator, and may be a hydraulic cylinder, a pneumatic cylinder, or the like.

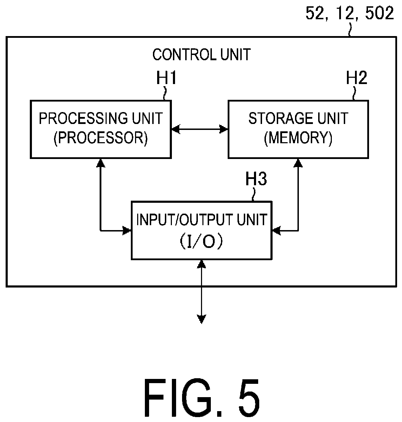

FIG. 5 is a diagram illustrating an example of a hardware configuration of the control unit 52 of the controller 5, the control unit 12 of the terminal 10, or the control unit 502 of the controller 500. In FIG. 5, the control unit 52, 12, 502 has a configuration of a general computer, and includes a processing unit (processor) H1, a storage unit (memory) H2, and an input/output unit (I/O) H3. The storage unit H2 includes a read only memory (ROM), a random access memory (RAM), a nonvolatile-RAM (NV-RAM), a solid state drive (SSD), and a hard disk drive (HDD).

The processing unit H1 executes processing based on data stored in the storage unit H2 or data input from the input/output unit H3 in accordance with a program stored in the storage unit H2. Data obtained by the processing is output from the input/output unit H3.

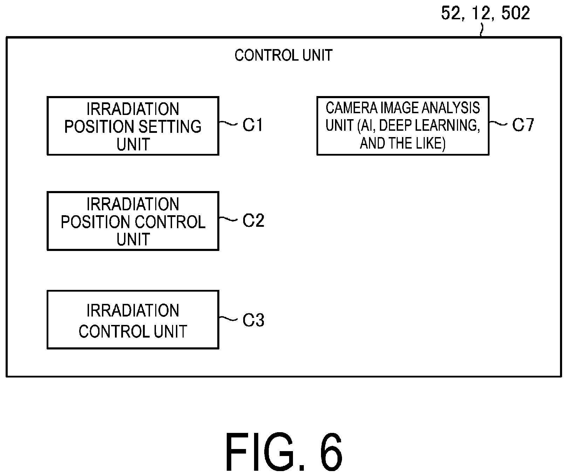

FIG. 6 is a diagram illustrating an example of a functional configuration the control unit 52 of the controller 5, the control unit 12 of the terminal 10, or the control unit 502 of the controller 500. In FIG. 6, the control unit 52, 12, 502 includes an irradiation position setting unit C1, an irradiation position control unit C2, an irradiation control unit C3, and a camera image analysis unit C7.

The irradiation position setting unit C1 has a function of setting a position or a range (a range is designated by a plurality of positions or the like) to be irradiated with ultraviolet rays for sterilization. The irradiation position control unit C2 has functions of controlling the vertical angle adjustment unit 3 and the horizontal angle adjustment unit 4 of the sterilizer 1 based on the setting information and controlling the position irradiated with ultraviolet rays. The irradiation control unit C3 has a function of turning ON and OFF the ultraviolet rays, and of changing the intensity of the ultraviolet rays (the intensity of the ultraviolet rays may or may not be changeable depending on the light sources), or changing the wavelengths of the ultraviolet rays (switching can be performed between a wavelength settable for human irradiation and a wavelength not settable for human irradiation but has a high sterilization effect) based on the setting information and the like.

The camera image analysis unit C7 has a function of acquiring the presence, position, and the like of the target of the sterilization such as a person or a pet from a camera image obtained by the camera 300, based on an artificial intelligence (AI) or reinforcement learning based learned model.

### Real Time Operation by User

In FIGS. 1 to 6, the user operates the terminals 10 and the like to change the status of the sterilizer 1 (1A, 1B), the irradiation status of the light source unit 21, and the orientation status of the light source unit 21. For example, when the user operates the terminal 10 to turn ON the light source unit 21, the controller 5 turns ON the light source unit 21 via the light source unit drive circuit 53, based on the irradiation control signal corresponding to the operation information corresponding to the turn ON. With the light source unit 21 turned ON, the sterilizer 1 irradiates the sterilization target space S with the ultraviolet rays UV in a radiating form, to sterilize the air in the sterilization target space S and an object in the sterilization target space S with the ultraviolet rays UV. When the user operates the terminal 10 to change the irradiation range of the ultraviolet rays UV in the sterilization target space S in the vertical direction, the controller 5 drives the first motor 32 via the first motor drive circuit 54 based on the drive control signal for the first motor 32 corresponding to the operation information corresponding to the adjustment of the vertical angle to rotate the light source unit 21 in the vertical direction. When the user operates the terminal 10 to change the irradiation range of the ultraviolet rays UV in the sterilization target space S in the horizontal direction, the controller 5 drives the second motor 42 via the second motor drive circuit 55 based on the drive control signal for the second motor 42 corresponding to the operation information corresponding to the adjustment of the horizontal angle to rotate the light source unit 21 in the horizontal direction. Thus, the user who wants to sterilize an object in the sterilization target space S rotates the light source unit 21 in the vertical direction and the horizontal direction, to move the irradiation range of the ultraviolet rays UV from the light source unit 21 to the object, and performs the sterilization.

As described above, in the sterilizer 1 (1A, 1B) according to the present embodiment, the light source unit 21 can be rotated in the horizontal direction and the vertical direction, so that the irradiation range of the ultraviolet rays UV for the sterilization target space S can be large compared with a case where the light source unit 21 cannot rotate. Thus, a wide range of the sterilization target space S can be sterilized, whereby the environment in the space can be easily improved.

The light source unit 21 emits the ultraviolet rays UV being electromagnetic waves having wavelengths of 190 nm to 230 nm. Thus, even if a person is present in the sterilization target space S and the person is irradiated with the ultraviolet rays UV from the light source unit 21, the impact of the emitted ultraviolet rays on the human body is extremely small. Therefore, even if there is a person in the sterilization target space S, the sterilization by the sterilizer 1 can be safely performed.

In the above description, the sterilizer 1 (1A, 1B) is operated by a manual operation by the user, but is not limited to being operated by the manual operation, and may be operated by automatic operation. For example, a sensor reacting to the movement of an object, such as a human sensor, for example, and having a following mode may be used. Under the following mode, the irradiation range of the ultraviolet rays UV changes to follow the movement of the object. The ultraviolet rays UV may be emitted in the irradiation direction while following a direction of location of a predetermined object, for example, a person. The control unit 52 may have a sterilization mode for an operation set in advance, and in the mode, a floor or a wall forming the sterilization target space S is entirely irradiated with the ultraviolet rays UV for example. The control unit 52 may irradiate the sterilization target space S with the ultraviolet rays UV, during predetermined time, for example, a time zone with absence of any person in the sterilization target space S.

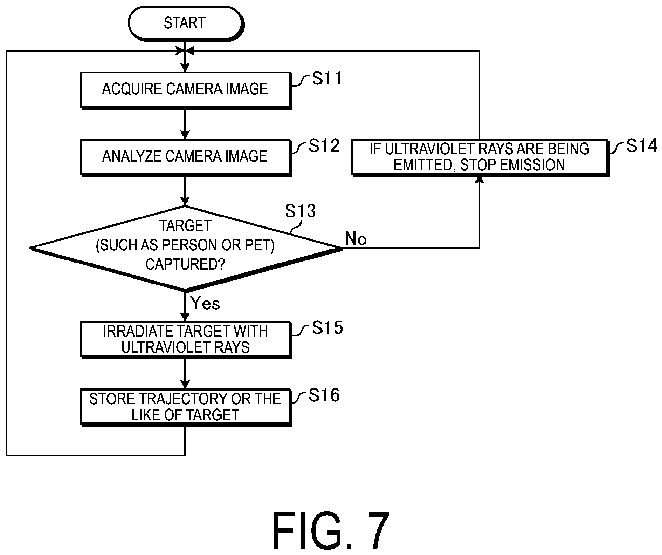

Real Time Ultraviolet Ray Irradiation based on Camera Image FIG. 7 is a flowchart illustrating an example of control by the control unit 52 of the controller 5, the control unit 12 of the terminal 10, or the control unit 502 of the controller 500. In FIG. 7, when processing starts, the control unit 52 or the like acquires a camera image from the camera 300 (FIG. 1) or the like (step S11), and analyzes the camera image with AI, deep learning, or the like (step S12).

The camera image analysis unit C7 (FIG. 6) outputs positions (center positions, contour positions, distances, or the like) of images recognized as a person, a pet, and the like from the camera image based on a learned model learned in advance for recognition of a person, a pet, or the like in a camera image including a large number of persons, pets, or the like. The positions are converted into directions (horizontal angle, vertical angle), and in the directions, the sterilizer 1 emits the ultraviolet rays.

Next, the control unit 52 or the like determines whether the target such as a person or a pet has been captured (Step S13), and upon determining no capture (No in step S13), stops the ultraviolet ray irradiation when the ultraviolet ray is being emitted (step S14), and returns to the acquisition of camera image (step S11).

When the target such as a person or a pet was determined to have been captured (Yes in step S13), the control unit 52 or the like emits the ultraviolet rays with the optical axis of the light source unit 21 directed to the target (step S15). When a person or a pet is irradiated with ultraviolet rays, wavelengths harmless to the human body or the like (for example, 222 nm) are used. In addition, the irradiation time and the irradiation intensity of the ultraviolet rays can be changed according to the distance to the target.

Next, the control unit 52 or the like stores the trajectory (movement path) of the target (step S16), and returns to the acquisition of camera image (step S11).

By such processing, the target such as a person or a pet can be automatically tracked and irradiated with ultraviolet rays to be sterilized.

### Ex-post Facto Ultraviolet Ray Irradiation based on Camera Image

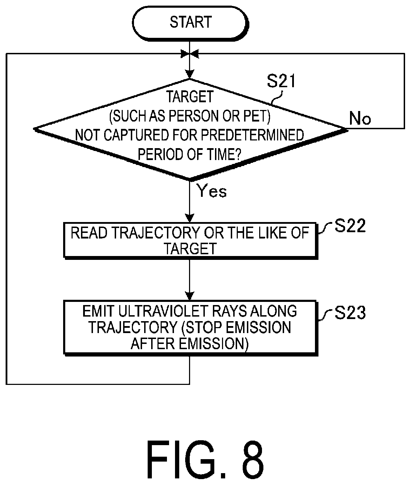

FIG. 8 is a flowchart illustrating another example of the control by the control unit 52 of the controller 5, the control unit 12 of the terminal 10, or the control unit 502 of the controller 500. In FIG. 8, when processing starts, the control unit 52 or the like determines whether the target such as a person or a pet has not been captured for a predetermined period of time (step S21), and repeats the determination, if the target was determined to have been captured (No in step S21).

When the target such as a person or a pet was determined to have not been captured for the predetermined period of time (Yes in step S21), the control unit 52 or the like reads information (stored in step S16 in FIG. 7) such as the trajectory (movement path) of the target having already been recorded (step S22).

Then, the control unit 52 or the like performs irradiation of ultraviolet rays along the read trajectory (step S23). In this case, since a person or a pet itself is not the irradiation target, wavelengths with a high sterilization effect (for example, wavelengths exceeding 230 nm) may be used for the ultraviolet rays emitted, or the irradiation time or the irradiation intensity may be increased. Then, the processing returns to the determination (step S21) on whether the target such as a person or a pet has not been captured for the predetermined period of time.

With such processing, a portion where a target such as a person or a pet is likely to have touched or passed through can be effectively sterilized, and the portion is difficult to be irradiated with the ultraviolet ray while the target is in the portion to cast a shadow.

The embodiment of the present invention has been described above, but the present invention is not limited to the embodiment described above, and various modifications are possible without departing from the spirit of the present invention.

As described above, the sterilization system according to the embodiment includes: a light source unit configured to emit ultraviolet rays in a radiating form; a horizontal angle adjustment unit configured to adjust a horizontal angle of the light source unit by causing rotation of the light source unit in a horizontal direction; and/or a vertical angle adjustment unit configured to adjust a vertical angle of the light source unit by causing rotation of the light source unit in a vertical direction; and a controller configured to capture a target of sterilization in a camera image, control the rotation caused by the horizontal angle adjustment unit and/or the vertical angle adjustment unit, and irradiate the target with the ultraviolet rays by the light source unit. Thus, a sterilization target space can be easily sterilized over a wide range.

Further, the controller captures the target in the camera image, based on an artificial intelligence or deep learning based learned model. Thus, the target can be accurately captured.

The controller stores the movement path of the target and irradiates the movement path with the ultraviolet rays from the light source unit. Thus, a portion difficult to be sterilized when the target exists can be sterilized.

When the target is a person or a pet, a wavelength not affecting the human body is used for the ultraviolet rays emitted from the light source unit. Thus, the target can be directly irradiated with ultraviolet rays safely.

For the ultraviolet rays from the light source unit, a wavelength providing a high sterilization effect is used without the influence on the human body. Thus, the sterilization can be performed more effectively.

Moreover, the present invention is not limited to the embodiment described above. A configuration obtained by appropriately combining the above-mentioned constituent elements is also included in the present invention. Further effects and modified examples can be easily derived by a person skilled in the art. Thus, a wide range of aspects of the present invention is not limited to the embodiment described above and may be modified variously.

### Reference Signs List

1, 1A, 1B Sterilizer, 21 Light source unit, 3 Vertical angle adjustment unit, 4 Horizontal angle adjustment unit, 5 Controller, 10 Terminal, 500 Controller, C1 Irradiation position setting unit, C2 Irradiation position control unit, C3 Irradiation control unit, C7 Camera image analysis unit

## Claims

1. A sterilization system comprising:
a light source unit configured to emit ultraviolet rays in a radiating form;
a horizontal angle adjustment unit configured to adjust a horizontal angle of the light source unit by causing rotation of the light source unit in a horizontal direction; and/or
a vertical angle adjustment unit configured to adjust a vertical angle of the light source unit by causing rotation of the light source unit in a vertical direction; and
a controller configured to capture a target of sterilization in a camera image, control the rotation caused by the horizontal angle adjustment unit and/or the vertical angle adjustment unit, and irradiate the target with the ultraviolet rays from the light source unit.

2. The sterilization system according to claim 1, wherein
the controller captures the target in the camera image, based on an artificial intelligence or deep learning based learned model.

3. The sterilization system according to claim 1 or 2, wherein
the controller stores a movement path of the target, and irradiates the movement path with the ultraviolet rays from the light source unit.

4. The sterilization system according to claim 1 or 2, wherein
when the target is a person or a pet, a wavelength not affecting the human body is used for the ultraviolet rays from the light source unit.

5. The sterilization system according to claim 3, wherein
for the ultraviolet rays from the light source unit, a wavelength providing a high sterilization effect is used without influence on the human body.

6. A sterilizer comprising:
a light source unit configured to emit ultraviolet rays in a radiating form;
a horizontal angle adjustment unit configured to adjust a horizontal angle of the light source unit by causing rotation of the light source unit in a horizontal direction; and/or
a vertical angle adjustment unit configured to adjust a vertical angle of the light source unit by causing rotation of the light source unit in a vertical direction; and
a controller configured to capture a target of sterilization in a camera image, control the rotation caused by the horizontal angle adjustment unit and/or the vertical angle adjustment unit, and irradiate the target with the ultraviolet rays from the light source unit.

7. A controller configured to perform for
a light source unit configured to emit ultraviolet rays in a radiating form, and
a horizontal angle adjustment unit configured to adjust a horizontal angle of the light source unit by causing rotation of the light source unit in a horizontal direction, and/or
a vertical angle adjustment unit configured to adjust a vertical angle of the light source unit by causing rotation of the light source unit in a vertical direction,
capturing of a target of sterilization in a camera image, controlling of the rotation caused by the horizontal angle adjustment unit and/or the vertical angle adjustment unit, and irradiating of the target with the ultraviolet rays from the light source unit.

8. A control method comprising, by a computer, executing for
a light source unit configured to emit ultraviolet rays in a radiating form, and
a horizontal angle adjustment unit configured to adjust a horizontal angle of the light source unit by causing rotation of the light source unit in a horizontal direction, and/or
a vertical angle adjustment unit configured to adjust a vertical angle of the light source unit by causing rotation of the light source unit in a vertical direction,
capturing of a target of sterilization in a camera image, controlling of the rotation caused by the horizontal angle adjustment unit and/or the vertical angle adjustment unit, and irradiating of the target with the ultraviolet rays from the light source unit.

9. A control program causing a computer to execute for
a light source unit configured to emit ultraviolet rays in a radiating form, and
a horizontal angle adjustment unit configured to adjust a horizontal angle of the light source unit by causing rotation of the light source unit in a horizontal direction, and/or
a vertical angle adjustment unit configured to adjust a vertical angle of the light source unit by causing rotation of the light source unit in a vertical direction,
capturing of a target of sterilization in a camera image, controlling of the rotation caused by the horizontal angle adjustment unit and/or the vertical angle adjustment unit, and irradiating of the target with the ultraviolet rays from the light source unit.
